# EUROPEAN PATENT SPECIFICATION

(11) **EP 0 839 036 B1**
(45) Date of publication and mention of the grant of the patent: **25.08.1999**
(21) Application number: 96924834.3
(22) Date of filing: 28.06.1996
(51) Int. Cl.: A61K 31/44

(54) **USE OF 1,4-DIHYDROPYRIDINE DERIVATIVES IN THE PREVENTION AND THERAPY OF ATHEROSCLEROTIC DEGRADATION OF ARTERIAL WALLS**
VERWENDUNG VON 1,4-DIHYDROPYRIDINDERIVATEN IN DER PRÄVENTION UND THERAPIE DER ATHEROSKLEROTISCHEN DEGENERATION VON ARTERIENWÄNDEN
UTILISATION DE DERIVES DE 1,4 DIHYDROPYRIDINE DANS LA PREVENTION ET LA THERAPIE DE LA DEGRADATION ATHEROSCLEREUSE DES PAROIS ARTERIELLES

(30) Priority: 14.07.1995 IT MI951513
(43) Date of publication of application: 06.05.1998
(73) Proprietor: RECORDATI S.A. CHEMICAL and PHARMACEUTICAL COMPANY, 6830 Chiasso (CH); RECORDATI INDUSTRIA CHIMICA E FARMACEUTICA S.p.a., 20148 Milano (IT)
(72) Inventor: SARTANI, Abraham, I-20020 Arese (IT); LEONARDI, Amedeo, I-20154 Milano (IT); TESTA, Rodolfo, I-20060 Vignate (IT)
(74) Representative: SERJEANTS
(86) International application number: EP9602872
(87) International publication number: WO9703669

(56) References cited:
- J.CARDIOVASC.PHARMACOL., vol. 19, no. SUPL.2, 1992, pages 8-16, XP000604014 PAULETTO P., ET AL.: "CALCIUM ANTAGONISTS AND VASCULAR SMOOTH MUSCLE CELLS IN ATHEROGENESIS"
- J.CARDIOVASC.PHARMACOL., vol. 19, no. SUPL 3, 1992, pages 29-31, XP000604015 SINZINGER H., ET AL.: "ANTIATHEROSCLEROTIC ACTIONS OF ISRADIPINE"
- CARDIOVASC.DRUGS THER., vol. 4, no. SUPL.5, August 1990, pages 1027-1031, XP000604409 KNORR A., ET AL.: "INFLUENCE OF NIFEDIPINE ON EXPERIMENTAL ARTERIOSCLEROSIS"
- J.HUMAN HYPERTENS., vol. 6, no. SUPL. 1, December 1992, pages 13-18, XP000604086 FLECKENSTEIN-GRÜN G., ET AL.: "PROTECTIVE EFFECTS OF VARIOUS CALCIUM ANTAGONISTS AGAINST EXPERIMENTAL ARTERIOSCLEROSIS"
- ACTA ANAESTHESIOL. SCAND., vol. 37, no. SUPL. 99, 1993, pages 33-37, XP000604080 LESLIE J.B.: "HAEMODYNAMICS AND TISSUE SPECIFITY WITH ISRADIPINE"
- J.CARDIOVASC. PHARMACOL., vol. 24, no. SUPL. 2, 1994, pages 85-91, XP000603993 BORCHARD U.: "CALCIUM ANTAGONISTS IN COMPARISON: VIEW OF THE PHARMACOLOGIST"
- DRUGS, vol. 48, no. SUPL. 1, 1994, pages 1-7, XP000603980 PAULETTO P., ET AL.: "IMPACT OF NIFEDIPINE ON VASCULAR SMOOTH MUSCLE CELL DIFFERENTIATION"
- PHARMACOL. RES., vol. 31, no. 314, March 1995 - April 1995, pages 251-254, XP000604033 GAVIRAGHI G., ET AL.: "RECENT DEVELOPMENTS IN THE USE OF CALCIUM ANTAGONISTS IN MYOCARDIAL PROTECTION"
- AM. J. HYPERTENS., vol. 7, no. 10(2), October 1994, pages 126-130, XP000604034 NAYLER W.: "NEW THERAPEUTIC TRENDS IN CALCIUM ANTAGONISM. ARE THEY MEANINGFUL?"
- J. CARDIOVASC. PHARMACOL., vol. 12, no. SUPL. 6, 1988, pages 29-35, XP000604025 WEINSTEIN D.B.: "THE ANTIATHEROGENIC POTENTIAL OF CALCIUM ANTAGONISTS"
- CLIN. INVEST. MED., vol. 10, no. 6, November 1987, pages 601-605, XP000604021 HENRY P.D.: "ANTI-ATHEROSCLEROTIC EFFECTS OF CALCIUM ANTAGONISTS. A BRIEF REVIEW"
- AM. J. MED., vol. 86, no. SUPL. 4(A), 17 April 1989, pages 27-32, XP000604027 WEINSTEIN D.B., ET AL.: "ANTIATHEROGENIC PROPERTIES OF CALCIUM ANTAGONISTS"

## Description

The invention relates to the use of 1,4-dihydropyridines to counter several processes which play a role in the development of atherosclerotic vascular lesions, such as myocytes proliferation and migration, cholesterol metabolism in macrophages and oxidative modification of low density lipoproteins. Beneficial effects on the above biological processes can be considered the basis for prevention of atherosclerotic degradation in the arterial walls of humans. The invention also relates to the use of 1,4-dihydropyridines in the manufacture of medicaments for preventing, arresting and reversing atherosclerotic degradation in the arterial walls of humans.

Arteriosclerosis, a generic term for thickening and hardening of the arterial wall, is responsible for very many deaths in the United States and other westernized societies. One type of arteriosclerosis is atherosclerosis, the disorder of the larger arteries that underlies most coronary artery disease, aortic aneurysm, and arterial disease of the lower extremities and also plays a major role in cerebrovascular disease. Atherosclerosis is by far the leading cause of death in the United States, both above and below age 65.

It is now recognized that atherosclerosis is a multifactorial process which, when leading to clinical sequelae, is based on extensive proliferation of migrated smooth muscle cells within the intima of the affected artery. The atherosclerotic plaque formation can be considered the result of three fundamental biological processes. These are:
1) migration and proliferation of intimal smooth muscle cells, together with variable numbers of accumulated macrophages and T-lymphocytes;
2) formation by the proliferated smooth muscle cells of large amounts of connective tissue matrix, including collagen, elastic fibres, and proteoglycans; and
3) accumulation of lipid, principally in the form of cholesteryl esters and free cholesterol within the cells as well as in the surrounding connective tissues.

In addition, a number of experimental reports suggest a key role for the oxidative modification of low density lipoproteins (LDL) in the early stages of atherosclerosis in humans, where hypercholesterolemia represents the major risk factor associated with the increased incidence of the disease. Available data suggest that LDL undergoes oxidative modification, and that oxidized LDL may promote atherogenesis by a number of mechanisms, including their enhanced uptake in tissue macrophages which leads to lipid accumulation and chemotactic activity for monocytes, and cytotoxicity to arterial wall endothelial cells.

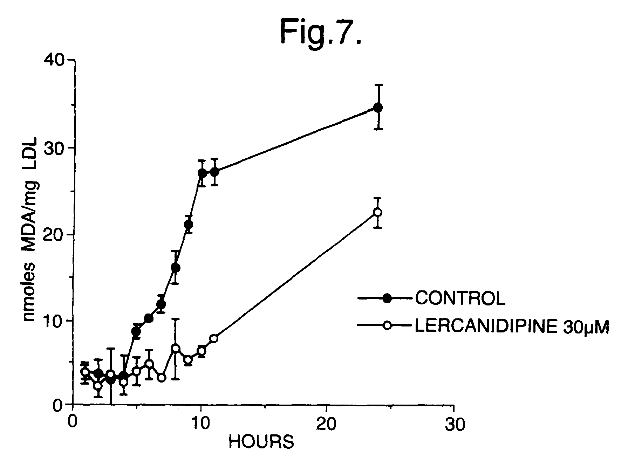

P. Pauletto et al [*J. Cardiovasc. Pharmacol*., 19 (Suppl. 2): S8-S16, 1992] teach that the majority of smooth muscle cells (SMCs) present in the atherosclerotic plaque show an "immature" type of myosin isoform expression. Nifedipine and nitrendipine in high doses are able to decrease the size of this particular SMC population and to prevent the development of atherosclerotic lesions in hypercholesterolemic rabbits. As indicated in Figure 7 of the article, nifedipine shows effect at 10⁻⁵M but no activity at 10⁻⁶M.

According to H. Sinzinger and P. Fitsch [*J. Cardiovasc. Pharmacol*., 19 (Suppl. 3): S29-S31, 1992], isradipine interferes with the three main mechanisms of human atherogenenesis, namely thrombus formation, cellular proliferation and lipid metabolism.

A.M. Knorr and S. Kazda (*Cardiovasc. Drugs Ther*., 4: 1027-1031, 1990) show that nifedipine is active in in vitro (SMC migration and proliferation; incorporation of cholesteryl esters into macrophages and SMC) and in vivo models (lowering of cholesterol and Ca⁺⁺ in the aortae of hypercholesterolemic rabbits; effects on endothelium and internal elastic lamina in hypertensive Dahl-S rats).

In *J. Human Hypertens*., 6 (Suppl.1): 513-518, 1992, G. Fleckenstein-Grün et al disclose that excessive calcium uptake into arterial walls plays a crucial role in the pathogenesis of atherosclerotic lesions. Amlodipine inhibits calcium accumulation and exerts protective effects against atherosclerotic lesions in Dahl-S rats. Amlodipine might be useful in prophylaxis of coronary artery lesions.

The inhibition by nifedipine of nonmuscle myosin isoform (typical of immature SMCs, which prevail in atherosclerotic lesions) expression and SMC differention is discussed by P. Pauletto et al in *Drugs*, 48 (Suppl.1): 1-7, 1994. These effects on immature SMC are confirmed in rabbits.

G. Gaviraghi et al in *Pharmacol. Res*., 31, 314: 251-254, 1995 review the use of dihydropyridines to reduce the formation of atheroma plaque. They point out the drawbacks of nifedipine, nisoldipine, isradipine and felodipine, but suggest that second generation dihydropyridines with slower onset and longer duration of action (e.g. lacidipine) act through a different mechanism and may overcome these drawbacks.

They also note that calcium entry blockers have been reported to be effective in reducing lesions in rabbit models of experimental atherosclerosis, but that all except isradipine require doses higher than are achievable in therapy.

P.D. Henry reviews the anti-atherosclerotic effects of calcium antagonists in *Clin*. *Invest*. *Med*., 10, 6: 601-605, 1987. Nifedipine inhibits the formation of atherosclerotic lesions in different animal models, at high doses. Isradipine was active at lower doses. In most studies the dihydropyridine activity was not associated with changes in plasma lipids and lipoproteins. Only one study with nicardipine at high doses decreased the cholesterol concentration in the d < 1.006 g/mL fraction. The effect of high doses of nicardipine on plasma lipids is not univocal since other authors failed to observe hypocholesterolemic effects. Nicardipine at low concentration inhibited the SMC migration induced by 12-HETE.

It has now surprisingly been found that certain 1,4-dihydropyridines, known from US Patent 4705797 for their coronary dilating and antihypertensive activity, are able to counteract many of the biological processes leading to atherosclerotic lesions and therefore can be used in humans to prevent and cure the atherosclerotic degradation of the arterial wall, hypercholesterolemia and various diseases caused by these, for example, ischemic heart diseases such as myocardial infarction and cerebrovascular diseases such as cerebral infarction and cerebral apoplexy. The compounds of the invention can also be useful for the inhibition of restenosis following percutaneous transluminal coronary angioplasty (PTCA) and for suppressing the progression of vascular hypertrophy associated with hypertension.

Particularly preferred among these 1,4-dihydropyridine derivatives are lercanidipine, its enantiomers, and pharmaceutically acceptable salts thereof. Lercanidipine is methyl 1,1,N-trimethyl-N-(3,3-diphenylpropyl)-2-aminoethyl 1,4-dihydro-2,6-dimethyl--4-(3-nitrophenyl)-pyridine-3,5-dicarboxylate.

On the one hand, the (S)-enantiomer and the racemate of lercanidipine, both being endowed with antihypertensive activity, can be used in patients in need of treatment for both hypertension and diseases related to atherosclerotic phenomena.

On the other hand, (R)-lercanidipine, being practically devoid of antihypertensive activity, can be used for treating conditions involving smooth muscle cell migration and proliferation without any concomitant cardiovascular effect. This enantiomer is therefore applicable to those patients for whom reduction of blood pressure would be undesirable.

The invention provides the use of a compound having the general formula I 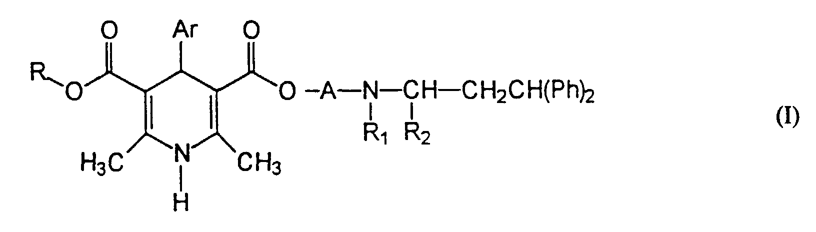 wherein
- Ph: represents a phenyl group,
- Ar: represents a 2-nitrophenyl, 3-nitrophenyl, 2,3-dichlorophenyl or benzofuran-4-yl group,
- A: represents a branched chain alkylene group having from 3 to 6 carbon atom,
- R: represents a straight or branched chain alkyl group having from 1 to 6 carbon atom, optionally mono-substituted by a alkoxy group having from 1 to 6 carbon atoms,
- R₁: represents a hydrogen atom, a hydroxy group or a alkyl group having from 1 to 4 carbon atom, and
- R₂: represents a hydrogen atom or a methyl group,
or of a salt, enantiomer, hydrate or solvate of such a compound, for the preparation of a medicament for the prevention, arrest or reversal of atherosclerotic degradation in the arterial walls of a patient.

The preferred 1,4-dihydropyridine derivatives I for use in the preparation of a medicament for administration to a patient, are lercanidipine and its (R)- and (S)-enantiomers.

Lercanidipine may be prepared by Hantzsch cyclization of methyl 3-aminocrotonate (1) with 1,1,N-trimethyl-N-(3,3-diphenylpropyl)-2-aminoethyl α-acetyl-3-nitrocinnamate (2) according to the method shown in Reaction Scheme 1 below and more fully described in US 4707797.

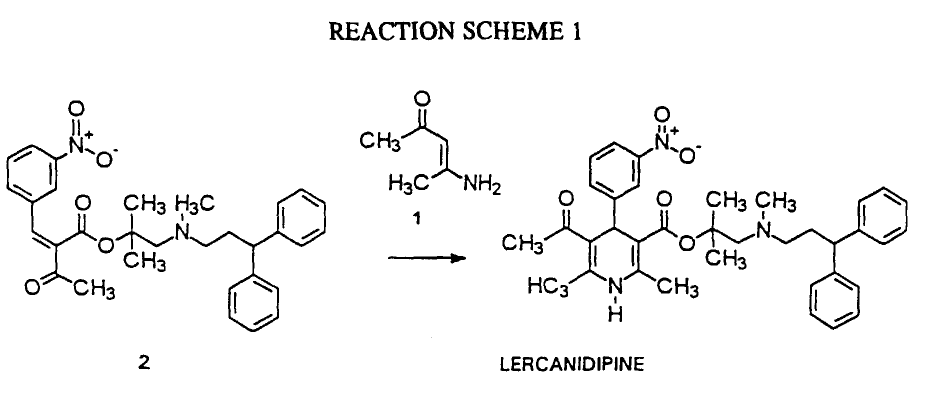

Lercanidipine may alternatively be prepared by esterification of 1,4-dihydro--2,6-dimethyl-5-methoxycarbonyl-4-(3-nitrophenyl)-pyridine-3-carboxylic acid (3) with 2,N-dimethyl-N-(3,3-diphenylpropyl)-1-amino-2-propanol (4) according to the method shown in Reaction Scheme 2 below and more fully described in Example 3 below.

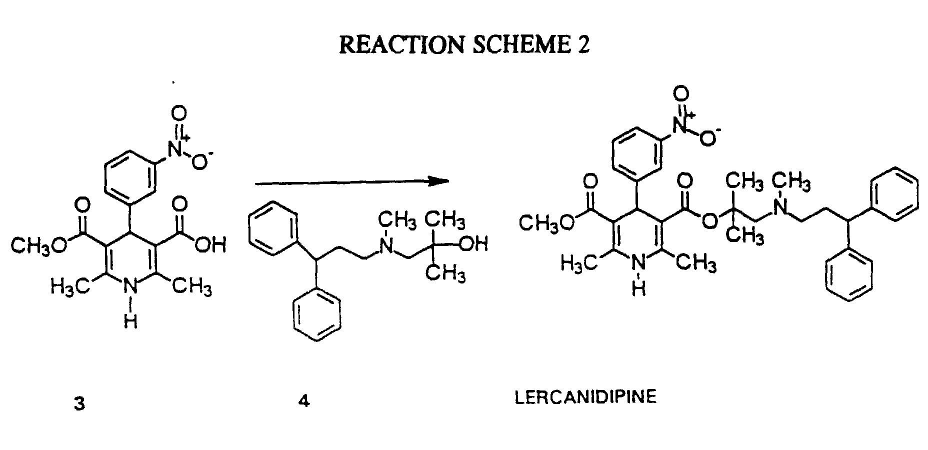

Lercanidipine enantiomers can be prepared by the esterification method reported in Reaction Scheme 2 above by using the appropriate homochiral acids which, when present in 1:1 ratio, constitute the above cited acid 3. These homochiral acids, hereinbelow for convenience called acid 5 [the (R)-enantiomer] and acid 6 [the (S)-enantiomer], can readily be prepared by resolution of the racemic acid 3 according to the methods reported by A. Ashimori et al., *Chem. Pharm. Bull*. 39, 108 (1991).

The esterification of Reaction Scheme 2 may be performed in the presence of a coupling agent, such as dicyclohexylcarbodiimide, N,N'-carbonyldiimidazole or diethyl cyanophosphonate, and optionally in the presence of a promoting agent, such as N-hydroxysuccinimide or 4-dimethylaminopyridine, in aprotic or chlorinated solvents, e.g. dimethylformamide or chloroform, at temperatures ranging from -10 to 140°C according to well known synthetic methods: Albertson, *Org. React*. 12, 205 (1982); Doherty et al., *J. Med. Chem*. 35, 2 (1992); Staab et al., *Newer Methods Prep. Org*. *Chem*. 5, 81 (1988); Ishihara, *Chem. Pharm. Bull*. 39, 3238 (1991).

Alternatively, lercanidipine enantiomers may be prepared by first reacting the acid 5 (or 6) with alkyl chloroformate in presence of a tertiary amine such as triethylamine, and then adding the intermediate (4) at 0-80°C. Optionally, a promoting agent such as 1-hydroxypiperidine may be added before the addition of the intermediate (4), see Albertson*, Org. React*. 12, 157 (1982)).

The lercanidipine enantiomers may be also prepared by conversion of the acid 5 (or 6) into the corresponding acyl halide using an inorganic acid halide, such as phosphorus pentachloride, oxalyl chloride, phosphorus trichloride, phosphorous oxychloride or thionyl chloride, in a chlorinated solvent, e.g. chloroform, dichloroethane, dichloromethane or 1,1,1-trichloroethane, optionally in the presence of a promoting agent such as dimethylformamide, at a temperature of from -10 to 85°C. The acyl halides may, but need not, be isolated before the addition of the intermediate (4).

The lercanidipine enantiomers, howsoever obtained, may be purified according to methods known in the art, either as bases (e.g. by column chromatography) or as salts (e.g. by reprecipitation or recrystallization). The above methods can also be used for all the other compounds of the general formula I.

According to the invention, the 1,4-dihydropyridine derivative I may be administered to the patient as such, or in the form of any of its pharmaceutically acceptable salts, hydrates or solvates. Preferred pharmaceutically acceptable acid addition salts include those formed with hydrochloric, sulphuric, maleic, succinic, citric, methanesulphonic and toluenesulphonic acids; they may be prepared from the free bases in conventional manner. Whatever the form (base, salt, hydrate or solvate), the active ingredient will usually be administered in admixture with a pharmaceutically acceptable carrier.

For oral administration, the 1,4-dihydropyridine derivatives may be incorporated with excipients and used in the form of tablets, troches, capsules, elixirs, suspensions, syrups, wafers, chewing gum and the like. These preparations should contain at least 0.5% of active compounds, but the amount of active ingredient may be varied depending upon the particular form and may conveniently be from about 5% to about 70% of the weight of the unit. The amount of active compound in such compositions is such that a suitable dosage will be obtained, although the desired dosage can be obtained by administering a plurality of dosage forms. The compounds of the invention may be administered at the oral dosage of 0.1 to 400 mg, the 1 to 200 mg dosage range being preferred. The tablets, pills, capsules, troches and the like may also contain, for example, the following ingredients: a binder such as microcrystalline cellulose, tragacanth gum or gelatin; an excipient such as starch or lactose; a disintegrating agent such as alginic acid, sodium starch glycolate, corn starch and the like; a lubricant such as magnesium stearate or hydrogenated castor oil; and a glidant such as colloidal silicon dioxide. Sweetening agents such as sucrose or saccharin can be included, as can flavouring agents such as peppermint, methyl salicylate or orange flavouring. When the dosage unit form is a capsule, it may contain, in addition to materials of the above type, a liquid carrier such as a fatty oil. Oral dosage units may contain various other materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes, colouring and flavours. Materials used in preparing these various compositions should be pharmaceutically pure and non-toxic in the amounts used.

For parenteral administration, the 1,4-dihydropyridine derivatives may be incorporated into a solution or suspension. These preparations should contain at least 0.1% of active compound, but the amount of active ingredient may vary between 0.5% and about 30% of the weight thereof. The amount of active compound in such compositions is such that a suitable dosage will be obtained. Preferably a parenteral dosage unit contains between 0.05 to 100 mg of active compound. The solutions or suspensions may also include the following components: a sterile diluent such as water for injection, saline solution, fixed oils, polyethylene glycols, glycerine, propylene glycol or other synthetic solvents; an antibacterial agent such as benzyl alcohol or methyl parabens; an antioxidant such as ascorbic acid or sodium bisulphite; a chelating agent such as ethylenediaminetetraacetic acid; a buffer such as an acetate, a citrate or a phosphate; and an agent for the adjustment of toxicity such as sodium chloride or dextrose. Parenteral multiple dose vials may be of glass or plastics material.

Dosage forms, additional ingredients and routes of administration contemplated herein include those disclosed in US 4089969 and US 5091182, both incorporated by reference in their entirety.

The following Examples illustrate the preparation of (R,S)-lercanidipine and the (S)-and (R)-enantiomers thereof.

### EXAMPLE 1

### (S)-(+)-methyl 1,1-N-trimethyl-N-(3,3-diphenylpropyl)-2-aminoethyl 1,4-dihydro--2,6-dimethyl-4-(3-nitrophenyl)-pyridine-3,5-dicarboxylate [(S)-Lercanidipine] hydrochloride hemihydrate

0.13 ml of thionyl chloride was added at -10°C to a stirred suspension of 0.54 g of (R)-1,4-dihydro-2,6-dimethyl-4-(3-nitrophenyl)-5-methoxycarbonyl-pyridine--3-carboxylic acid in 2.9 ml of anhydrous dichloromethane and 0.75 ml of anhydrous dimethylformamide kept under nitrogen atmosphere and sheltered from direct light. After 1 hour at 0°C, a solution of 0.48 g of 2,N-dimethyl-N-(3,3-diphenyl-propyl)--1-amino-2-propanol (prepared as described in US 4705797) in 1 ml of dichloromethane was added at -5°C. After stirring for 3 hours at 0°C and standing overnight at 20-25°C, the solvent was evaporated off in vacuo and the residue was dissolved in 20 ml of ethyl acetate. The organic phase was washed sequentially with brine (4 ml), 10% aqueous sodium carbonate solution (5 x 4 ml), brine (4 ml), 1N hydrochloric acid (5 x 5 ml), brine (4 ml), 10% aqueous sodium carbonate solution (2 x 5 ml) and finally with brine (4 ml). The organic phase was dried over anhydrous sodium sulphate and evaporated to dryness in vacuo. The residue was purified by flash chromatography on a silica gel column eluting with petroleum ether:acetone 85:15. The unitary TLC fractions (Petroleum ether:acetone 7:3 by volume and chloroform:5N methanolic ammonia 99:1.1 by volume) were evaporated to give a residue which was dissolved in 75 ml of diethyl ether containing 3% of acetone. After filtration the solution was acidified with 3N ethereal hydrogen chloride and the precipitate was collected by suction and dried at 78°C/15 mmHg to give 0.66 g of the title compound.
M.p. 115-125°C; [α]_{D}²⁵= + 70.56° (MeOH; c = 0.981).

| Elemental analysis % for C₃₆H₄₁N₃O₆.HCl.0.5 H₂O | | | | | |
|---|---|---|---|---|---|
| Found: | C, 65.47; | H, 6.57; | N, 6.29; | Cl, 5.32; | H₂O, 1.68 |
| Calc.: | C, 65.79; | H, 6.60; | N, 6.39; | Cl, 5.39; | H₂O, 1.37 |

¹H-NMR Spectrum of the base at 200 MHz (CDCl₃, δ):

| | | |
|---|---|---|
| 8.10 | (m, 1H) | nitrophenyl, 2-CH |
| 7.97 | (m,1H) | nitrophenyl, 4-CH |
| 7.62 | (m,1H) | nitrophenyl, 6-CH |
| 7.33 | (dd, 1H) | nitrophenyl, 5-CH |
| 7.29-7.10 | (m, 10H) | aromatic H atoms of CH(Ph)₂ |
| 5.79 | (bs, 1H) | pyridine, NH |
| 5.05 | (s, 1H) | pyridine, 4-CH |
| 3.92 | (t, 1H) | CH(Ph)₂ |
| 3.63 | (s, 3H) | COOCH₃ |
| 2.57 | (m, 2H) | OC(CH₃)₂CH₂N |
| 2.40-2.23 | (m, 2H) | N(CH₃)CH₂CH₂ |
| 2.33-2.27 | (2s, 6H) | pyridine, 2-CH₃, and 6-CH₃ |
| 2.19-2.09 | (m, 2H) | N(CH₃)CH₂CH₂ |
| 2.17 | (s, 3H) | NCH₃ |
| 1.35-1.31 | (2s, 6H) | OC(CH₃)₂CH₂N |

### EXAMPLE 2

### (R)-(-)-methyl 1,1-N-triethyl-N-(3,3-diphenylpropyl)-2-aminoethyl 1,4-dihydro--2,6-dimethyl-4-(3-nitrophenyl)-pyridine-3,5-dicarboxylate [(R)-Lercanidipine] hydrochloride hemihydrate

The title compound was obtained by the method described in Example 1 for its (S)-enantiomer, but using the (S)-enantiomer of 1,4-dihydro-2,6-dimethyl--4-(3-nitrophenyl)-5-methoxycarbonyl-pyridine-3-carboxylic acid instead of the (R)-enantiomer.
M.p. 115-120°C, [α]_{D}²⁵ = -70.88 (MeOH, c = 0.975).

| Elemental analysis % for C₃₆H₄₁N₃O₆ .HCl .H₂O | | | | | |
|---|---|---|---|---|---|
| Found: | C, 64.93; | H, 6.62; | N, 6.24; | Cl, 5.41; | H₂O, 2.50 |
| Calc.: | C, 64.90; | H, 6.60; | N, 6.31; | Cl, 5.32; | H₂O, 2.70 |

The ¹H-NMR spectrum of the base in CDCl₃ was exactly the same as that reported in Example 1 for the (S)-enantiomer.

### EXAMPLE 3

### Methyl 1,1-N-trimethyl-N-(3,3-diphenylpropyl)-2-aminoethyl 1,4-dihydro--2,6-dimethyl-4-(3-nitrophenyl)-pyridine-3,5-dicarboxylate [Lercanidipine] hydrochloride

45.8 g (0.385 mole) of thionyl chloride was dropped over a period of 15 minutes into a stirred mixture comprising 116.2 g (0.35 mole) of 2,6-dimethyl-5-methoxycarbonyl--4-(3-nitrophenyl)-1,4-dihydropyridine-3-carboxylic acid (3), prepared as described in DE 2847237, 645 ml of anhydrous dichloromethane and 160 ml of anhydrous dimethylformamide, maintained in a nitrogen atmosphere at between -4°C and +1 °C. The mixture was kept at the same temperature under stirring for 1 hour, and then 104.1 g (0.35 mole) of 2,N-dimethyl-N-(3,3-diphenylpropyl)-1-amino-propanol (4), prepared as described in US 4705797, dissolved in 105 ml of anhydrous dichloromethane was added dropwise over a period of 15 minutes at -10°C to 0°C.

After stirring for three hours at 0°C and standing overnight at room temperature, the solvent was evaporated off under vacuum and the residue was dissolved in 3500 ml of ethyl acetate. The organic phase was washed sequentially with brine (700 ml), 10% aqueous sodium carbonate solution (5 x 700 ml), brine (700 ml), 1N hydrochloric acid (5 x 700 ml) and finally with brine (700 ml). The organic phase was dried over anhydrous sodium sulphate for 30 minutes, and then filtered, shaken with 23 g of charcoal ad filtered again. The volume of the solution was reduced to about 1 litre by evaporation under vacuum and then, after standing for 24 hours at from 0°C to 5°C, the crystals were collected by suction filtration and recrystallized from 99% ethanol to give 179.5(78%) of the title compound. m.p. 186-188°C.

### PHARMACOLOGICAL DATA

In the drawings:
Figure 1 is a graphical representation of the effect of lercanidipine and its enantiomers on [³H]-thymidine incorporation into myocetes of rat smooth muscle cells,
Figure 2 is a graphical representation of the ability of lercanidipine and its enantiomers to interfere with the migration of arterial myocetes,
Figure 3 is a graphical representation of the ability of lercanidipine and its enantiomers to inhibit the enzyme ACAT and cholesterol esterification induced by AcLDL in mouse peritoneal macrophage,
Figure 4 is a graphical representation of the concentration-dependent effect of lercanidipine and its enantiomers on cholesterol esterification in cholesterol ester loaded macrophages,
Figures 5 and 6 are graphical representations of the effect of lercanidipine and its enantiomers on cell-mediated oxidation of LDL, and
Figure 7 is a time course plot of cell-mediated oxidation and the effects of lercanidipine after incubation.

### Effects on arterial myocyte migration and proliferation

Animal models of vascular injury have shown that an arterial lesion is followed by proliferation of the medial myocytes, many of which migrate into the intima and further proliferate to form a neointimal lesion. The causes of these events are not completely understood. Recent findings have elucidated that myocytes make up approximately 90-95% of the cellular population of the atherosclerotic lesion in young adults and compose an average of 50% of the advanced atherosclerotic plaque. In addition, vascular myocytes contribute to the lesion by synthesis of extracellular matrix and they can accumulate lipids and become foam cells. Thus, the elucidation of the factors affecting these phenomena affords new entry points for selective interference and inhibition of the process of atherogenesis.

Migration of myocytes was investigated for the present invention using rat aortic smooth muscle cells in the presence of fibrinogen as chemotactic factor, whereas for studies on their proliferation rat and human cells were used. Cell count and [³H]-thymidine incorporation were used to evaluate myocytes growth. The methodology was as follows:

Myocytes were cultured from the intima-medial layer of the aortas of male Sprague-Dawley rats (200-250 g). Cells were grown in monolayers at 37°C in a humidified atmosphere of 5% CO₂ in Eagle's minimum essential medium supplemented with 10% (v/v) fetal calf serum, 100 U/ml penicillin, 0.1 mg/ml streptomycin, 20 mM tricine buffer and 1% (v/v) non-essential amino acid solution. The medium was changed every third day. Cells were used between the 4th and 10th passage. Cell viability was timely assessed by tripan blue exclusion. Myocytes were identified for growth behaviour, morphology and using monoclonal antibody specific for a-actin, the actin isoform typical of myocytes. Human vascular myocytes (A 617 from human femoral artery) were grown in the same culture conditions. Cells were seeded at various densities for rat (2 x 10⁵) and human (5 x 10⁴) myocytes/petri dish (35 mm), and incubated with Eagle's minimum essential medium supplemented with 10% fetal calf serum. Twenty-four hours later the medium was changed to one containing 0.4% fetal calf serum to stop cell growth, and the cultures were incubated for 72 hours. At this time (time 0) the medium was replaced by one containing 10% fetal calf serum in the presence or absence of known concentrations of the tested compounds and the incubation was continued for further 72 hours at 37°C. At time 0, just before the addition of the substances to be tested, a sample from each petri dish was used for cell counting. Cell proliferation was evaluated by cell count after trypsinization of the monolayers using a Coulter Counter model ZM. Cell viability was assessed by tripan blue exclusion, and found to be higher than 95% at the drug concentrations used. The results are shown in Table 1.

**TABLE 1**

| Myocytes growth inhibition measured by cell count | | | | | |
|---|---|---|---|---|---|
| Myocetes from | LE IC₅₀(µM) | (R)-LE IC₅₀(µM) | (S)-LE IC₅₀(µM) | NI IC₅₀(µM) | LA IC₅₀(µM) |
| SD Rat | 31.2 | 30.7 | 33.3 | 38.8 | 23.6 |
| SHR Rat | 14.1 | 9.2 | 16.8 | n.t. | n.t. |
| WK Rat | 16.4 | 15.3 | 20.7 | n.t. | n.t. |
| Human | 25.3 | n.t. | n.t. | n.t. | n.t. |
| LE = Lercanidipine NI = Nicardipine LA = Lacidipine IC₅₀ = concentration required to inhibit cell growth by 50% SD = Sprague Dawley* SHR = Spontaneously Hypertensive* WK = Wistar Kyoto* n.t. = not tested * = from Charles River, Calco, Italy | | | | | |

Lercanidipine and its enantiomers decreased the rat and human myocyte proliferation in a concentration-dependent manner, as shown in Table 1, and showed practically the same potency as the reference 1,4-dihydropyridines tested. It has to be emphasized that lercanidipine (and its enantiomers) proved active on the cells from all the species investigated, in particular in humans.

In another set of experiments, synchronization of myocytes to the G₀/G₁ interphase of the cell cycle was accomplished by incubating logarithmically growing cultures (= 3 x 10⁵ cells/plate) for 96-120 hours in a medium containing 0.4% fetal calf serum. Quiescent cells were incubated for 20 hours in a fresh medium with 10% fetal calf serum in the presence of the tested drugs. Cell proliferation was then estimated by nuclear incorporation of [³H]thymidine, incubated with cells (1 µCi/ml medium) for 2 hours. Radioactivity was measured with Filter-Count scintillation cocktail.

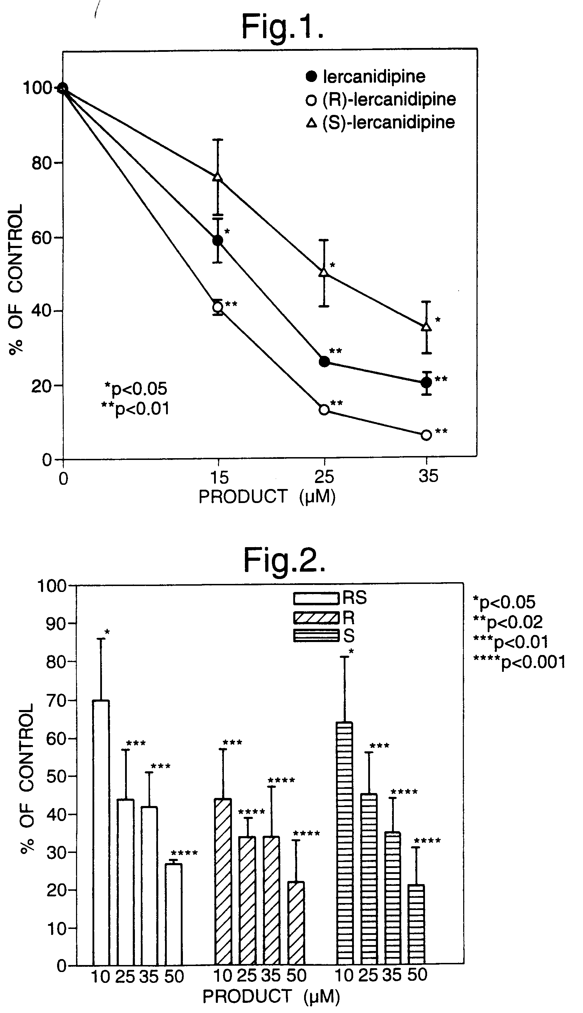

The results are shown in Figure 1, and confirm the high potency of lercanidipine and its enantiomers in inhibiting cell growth.

Migration of rat myocytes was examined using a 48-well micro chemotaxis chamber (Neuro-Probe, USA). Freshly trypsinized myocytes were suspended in a medium supplemented with 5% fetal calf serum (assay medium). The lower wells, containing 27 µl of the assay medium that included fibrinogen (600 µg/ml) as chemotactic agent, were covered with a polyvinylpyrrolidone-free polycarbonate filter (8 µM pore size). 50 µl of the cell suspension (1 x 10⁶ cells/ml) were placed in the upper compartment with the tested compounds. Incubation was carried out for 5 hours at 37°C in an atmosphere of 95% air and 5% CO₂. After incubation the filter was removed from the chamber and non-migrated cells were scraped from the upper surface, and the filters washed with phosphate buffered saline three times. The filters were stained with Diff-Quik (Merz-Dade AG, Switzerland). The number of myocytes per 100 x high power field that had migrated to the lower surface of the filters was determined microscopically. Six high power fields were counted per sample and the results were averaged.

The results are shown in Figure 2, and demonstrate the ability of lercanidipine and its enantiomers to interfere with the migration of arterial myocytes. All the tested compounds were able to inhibit myocytes migration in a dose-dependent manner with the (R)-enantiomer showing the most pronounced effect.

### Effects on cholesterol metabolism in mouse peritoneal macrophages

The atheroma contains two main cell types, macrophages and smooth muscle cells. Macrophages are derived from circulating monocytes and are the main lipid-loaded cells in the lesions. The mechanism by which they accumulate lipoprotein cholesterol and develop into foam cells depends mainly upon receptor-mediated processes, involving the so called "scavenger receptor" that recognizes chemically and biologically modified LDL, such as acetyl LDL (AcLDL) and oxidized LDL. The scavenger receptor, unlike the LDL receptor, is not subject to feed-back regulation and the result is a massive accumulation of cholesterol in cells. Cholesterol accumulates in macrophages in esterified form by a process involving the enzyme acyl-coenzyme A-cholesterol acyltransferase (ACAT) which catalyzes the cholesterol esterification in cytoplasm. Only free cholesterol can be removed from macrophages.

Cholesterol esterification induced by AcLDL in mouse peritoneal macrophages was investigated as follows. Mouse peritoneal macrophages were obtained by peritoneal lavage from mice (Balb/c Charles River, Calco, Italy) three days after intraperitoneal injection of thioglycolate. Cells (2-3x10⁶) were plated in 35 mm wells with Dulbecco's minimum essential medium containing 10% fetal bovine serum. After 3 hours, the dishes were washed to eliminate unattached cells and maintained in Dulbecco's minimum essential medium plus 10% fetal bovine serum for 24 hours before use. After cell plating, experiments were performed at 37°C in serum free Dulbecco's minimum essential medium containing 0.2% essentially fatty acid-free bovine serum albumin plus the indicated additions. Human LDL (d=1.019-1.063 g/ml) were isolated from plasma of healthy volunteers by sequential ultracentrifugation (Beckman L5-50, Palo Alto, CA). For acetylation, LDL were dialysed against 0.15 M NaCl, pH 7.4, diluted with an equal volume of saturated sodium acetate and treated with acetic anhydride. For [¹²⁵I]AcLDL, lipoproteins were labelled with sodium [¹²⁵I]iodide desalted by gel filtration on Sephadex G-25 eluted with phosphate buffered saline. Specific activity was 100-200 cpm/ng protein. Trichloroacetic acid non precipitable radioactivity was below 2% of total. All lipoproteins were sterile filtered. Cells were incubated for 24 hours with the tested compounds. After substitution of the medium with an identical one, incubation was continued for additional 24 hours. During this second incubation [¹²⁵I]AcLDL were added (50 µg/ml). Cholesterol esterification was measured after addition of [1-¹⁴C] oleic acid (0.68 mCi sample) complexed with bovine serum albumin during the last 1 or 2 hours of incubation and subsequent determination of radioactivity associated with cellular cholesteryl esters. Where indicated cells were enriched with cholesterol by 24 hours incubation with 50 µg/ml of AcLDL before drug addition and experimental determinations.

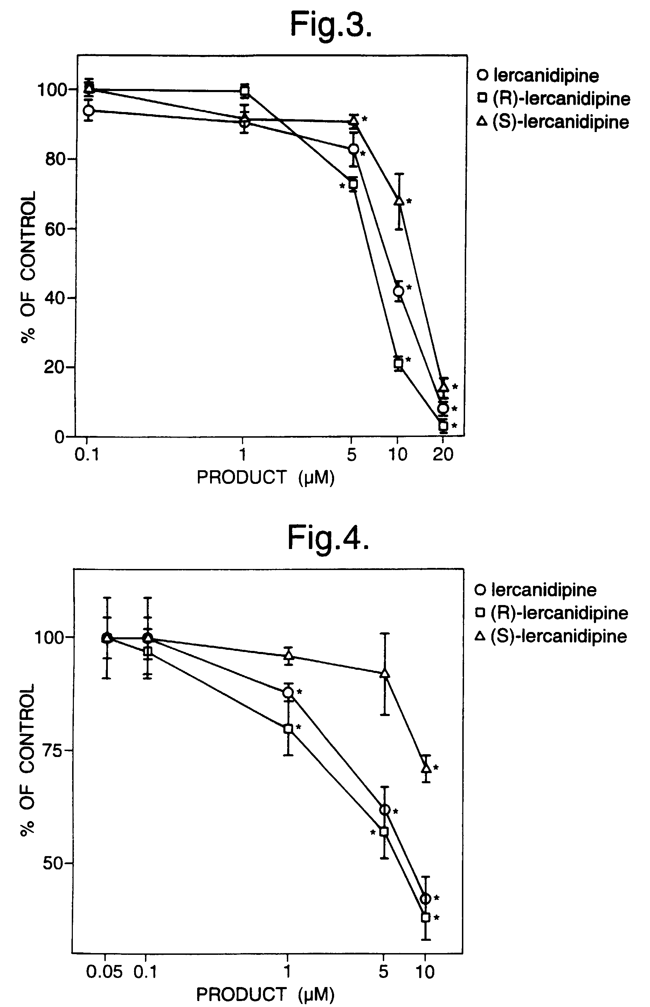

Lercanidipine and its enantiomers proved able to inhibit, in a concentration dependent way, up to 90% of the formation of esterified cholesterol induced by AcLDL in mouse peritoneal macrophage (in other words the esterifying effect of enzyme ACAT). The 1C₅₀ values for lercanidipine and enantiomers ranged from 8 to 15 µM, as shown in Figure 3, the (R)-enantiomer being the most active compound.

Another set of experiments was performed to evaluate the effect of lercanidipine on cholesterol esterification in macrophages loaded with cholesterol ester before the addition of the compound, this condition being the same as in foam cells. Cells are loaded with cholesteryl esters by exposure for 24 hours to a medium containing 50 µg acetyl LDL. The results, in Figure 4, show that lercanidipine inhibited cholesterol esterification up to 70% in a concentration dependent manner with an IC₅₀ value of about 7 µM. The (R)-enantiomer of lercanidipine proved slightly more potent than the racemate and (S)-lercanidipine was the least potent among the compounds tested.

Finally, it was proved that lercanidipine and its enantiomers at 5 µM did not impair the capability of the macrophages to hydrolyze the esterified cholesterol stored in the cytoplasm. These experiments were performed by incubating cells preloaded with [³H]cholesterol in the presence of the specific ACAT inhibitor S-58035. The blockade of intracellular re-esterification of cholesterol allowed the assessment of the ability of cells to hydrolyse the accumulated cholesterol esters. Addition of lercanidipine and its enantiomers did not influence the cellular hydrolytic activity as documented by the values of radioactivity in the esterified cholesterol fraction.

[1,2-³H]Cholesterol is included in all loading media at a concentration of 0.5 µCi/ml. After 24 hours loading period, during which time the radiolabeled cholesterol is incorporated and esterified, the cell monolayers are washed and incubated an additional 24 hours in medium containing 0.1% bovine serum albumin to allow the intracellular pools of labelled cholesterol to equilibrate to the same specific activity. To quantitate cholesteryl ester hydrolysis, the loaded cells are incubated for up to 24 hours in Dulbecco's minimum essential medium containing drugs, 0.1% bovine serum albumin, and the compound S-58035, an inhibitor of acyl-coenzyme A-cholesterol acyltransferase. The inhibition of acyl-enzyme A-cholesterol acyltransferase prevents the reesterification of any free cholesterol generated by cholesteryl ester hydrolysis and thus allows assessment of the activity of the hydrolase. The hydrolysis of the cholesteryl esters is quantitated by determining the decrease of radiolabeled cholesteryl esters [E.H. Harrison et al., *J. Lipid. Res*. 31, 2187 (1990)]. After the indicated incubations, cells were washed With phosphate buffered saline and extracted with hexane:isopropanol (3:2 v/v). Media were extracted with chloroform:methanol (2:1 v/v). After solvent removal, free and esterified cholesterol were partitioned by TLC (isooctane:diethyl ether:acetic acid, 75:25:2 by volume). Cholesterol mass or radioactivity of the spots were determined by an enzymatic method (Boehringer Mannheim, Germany) [F. Bernini et al., *Atherosclerosis* 104, 19 (1993)] or by liquid scintillation counting (Lipoluma Lumac, Lidgraf, The Netherlands) respectively. The results are given in Table 2.

**TABLE 2**

| Effect of lercanidipine and its enantiomers on cholesteryl ester hydrolysis in macrophages | |
|---|---|
| | % of cholesteryl ester |
| AcLDL 50 µg/ml | 31 ± 0.8 |
| AcLDL 50 µg/ml + S-58035 1 µg/ml | 15 ± 0.5 |
| AcLDL 50 µg/ml + S-58035 1 µg/ml + 5x10⁻⁶ M LE | 12 ± 1.2 |
| AcLDL 50 µg/ml + S-58035 1 µg/ml + 5x10⁻⁶ M (S)-LE | 14 ± 0.4 |
| AcLDL 50 µg/ml + S-58035 1 µg/ml + 5x10⁻⁶ M (R)-LE | 16 ± 2.1 |

### Effects on LDL oxidation

Experimental reports suggest a key role for the oxidative modification of LDL in the early stages of atherosclerosis in humans. Available data suggest that LDL undergoes oxidative modifications in vivo and that oxidatively modified LDL (Ox-LDL) may induce atherogenesis by a number of mechanisms, including its enhanced uptake in tissue macrophages (via the scavenger receptor pathway) which leads to lipid accumulation, and chemotactic activity for monocytes, and cytotoxicity to arterial wall endothelial cells.

Lercanidipine antioxidant capacity was evaluated by incubating LDL With 20 µM Cu⁺⁺ in the presence or absence of different concentrations of the tested compound (0.01 µM - 50 µM). LDL oxidation Was followed by monitoring conjugated diene formation at 234 nm. The experimental conditions were as follows. LDL (d = 1.019-1.063) were isolated from human pooled plasma by sequential ultracentrifugation at 4°C and 40,000 rpm in a 50Ti rotor, using a L5-50 ultracentrifuge (Beckman, Palo Alto, CA). LDL were then dialyzed against 0.15 M NaCl containing 0.01% ethylenediaminetetraacetic acid pH 7.4, sterilized by filtration through a 0.2 µM millipore filter and kept at 4°C under nitrogen in the dark until use (up to 3 weeks). Before use LDL were dialysed against ethylenediaminetetraacetic acid free phosphate buffered saline pH 7.4 on Sephadex G-25 columns (PD-10, Pharmacia Fine Chemicals, Uppsala, Sweden) and then LDL were filtered through a sterile 0.22 µM filter. LDL in phosphate buffered saline (50 µg lipoprotein protein/ml) were oxidized by incubation at 25°C with 20 µM CuSO₄ for 3 hours. The lercanidipine solution was prepared as a 10⁻² M stock solution in methanol and added as ethanol solution (maximum 1% v/v) prior to addition of copper. The effect of lercanidipine on the oxidation of LDL was determined by continuous monitoring the conjugated diene formation by recording the increase in the absorbance at 234 nm at 5 min intervals during a three hours period, against a phosphate buffered saline blank, using a UV spectrophotometer (Beckman DU 640) with continuous reading with a automatic 6-cell changer. The lag time of the onset of oxidation was calculated as the intercept between the line of the maximum slope of the propagation phase and the baseline where absorbance was that at time 0. The results are shown in Table 3.

**TABLE 3**

| Effect of lercanidipine on the lag time of LDL oxidation | |
|---|---|
| Lercanidipine µM | Lag time |
| 0 | 46.5 ± 4.6 |
| 0.5 | 45.1 ± 3.7 |
| 1.0 | 49.8 ± 4.7 |
| 2.5 | 53.6 ± 3.3* |
| 5.0 | 73.2 ± 4.8† |
| 10.0 | 112.7 ± 5.2† |

| | |
|---|---|
| * P < 0.05; | |
| † P < 0.01 | |

As Table 3 shows, 10 µM lercanidipine racemate doubled the lag-phase of LDL oxidation: the effect of the compound was concentration-dependent. The activity of the enantiomers was comparable to that of the racemate.

The antioxidant capacity of lercanidipine and its enantiomers was investigated also on cell-mediated oxidation. This was evaluated by incubating ethylenediaminetetraacetic acid free LDL under sterile conditions With 5 µM Cu⁺⁺ (100 µg Apo B/ml) in the presence of J774 cells or alternatively with EAhy-926 cells. Oxidation was blocked after 22 hours incubation by adding butylated hydroxytoluene to the medium (final concentration 40 µM) dissolved in ethanol. The extent of lipid peroxidation was measured by determining the percentage inhibition of aldehydic breakdown products using the thiobarbituric acid assay [A.N. Hanna et al., *Biochem. Pharmacol*. 45, 753 (1993)]. Briefly, to 0.250 ml of the incubated samples, 0.750 ml of trichloroacetic acid (0.20% w/v) was added followed by 0.750 ml of thiobarbituric acid (0.67% w/v). The samples were heated at 100°C for 20 min, followed by cooling and centrifugation. Malondialdehyde equivalents were calculated using 1,1,3,3-tetramethoxy-propane as standard.

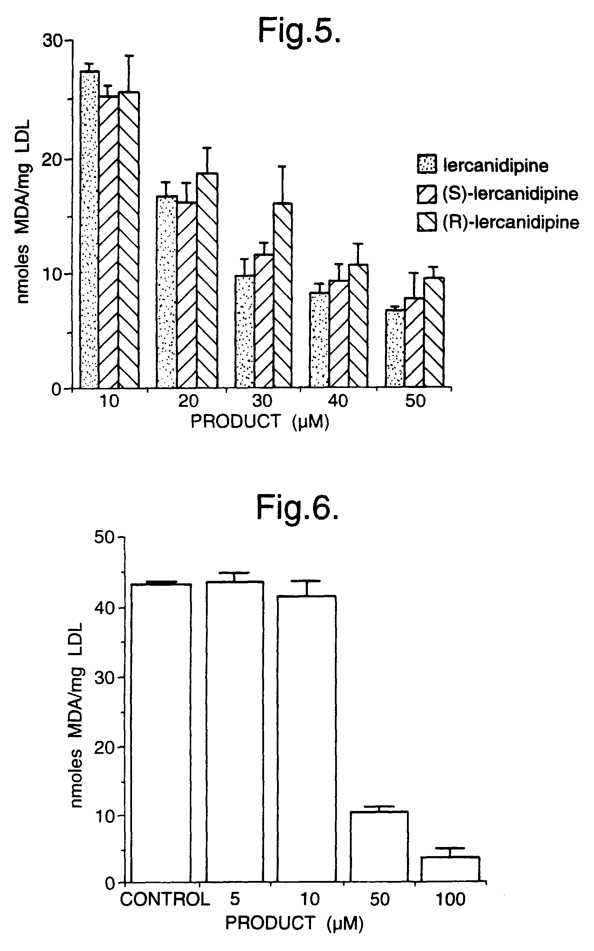

The results in Figure 5, obtained with ATCC TIB 67 J774A.1 cells, show that lercanidipine and its enantiomers were effective in reducing LDL oxidation. The results in Figure 6, obtained with EAhy 926 cells which share many of the properties of endothelial cells, show that lercanidipine reduced the oxidation of LDL in a range between 10 and 100 µM.

In the above experiments of cell-mediated oxidation the effects of lercanidipine were investigated after 22 hours incubation. In order to investigate the reason for the lower potency shown by lercanidipine in these conditions, the extent of lipid peroxidation in the presence of 30 µM lercanidipine was checked at different times by withdrawing samples of the incubation medium and measuring the oxidation compounds as above. The results are shown in Figure 7, and it can clearly be seen that 30 µM lercanidipine exerted a very high inhibition of lipid oxidation after 10 hours incubation. This result supports the view that lercanidipine at appropriate time could be as potent on cell-mediated LDL oxidation as it is on Cu⁺⁺-mediated oxidation.

Lercanidipine proved the most potent 1,4-dihydropyridine so far tested in these assays, its potency being one order of magnitude higher than that of lacidipine, the most potent in this experimental setting among the 1,4-dihydropyridine compounds previously known.

### Effects on blood pressure in hypertensive dogs

The antihypertensive effects of oral assumption of lercanidipine and its enantiomers were tested in renal hypertensive dogs.

Male beagle dogs weighing 12-13 kg, aged 1-3 years (Nossan Allevamenti, Italy) were used. Chronic sustained hypertension was induced by bilateral renal artery constriction, according to the Goldblatt method "two-kidney, two clip hypertension". Under barbiturate anaesthesia (35 mg/kg iv.) during two different surgical interventions one month apart from each other, both renal arteries were clipped with original renal silver clips and narrowed by about 60-70 %. After two months from the last intervention, an experimental renal hypertension was produced and the animals were suitable for the implantation of a catheter. Under sodium pentobarbital anaesthesia (35 mg/kg iv.), in sterile conditions, the dogs were catheterized by inserting an indwelling cannula (PE 200 Clay Adams) into the ascending aorta through the right common carotid artery. The catheter was subcutaneously exteriorized at the back of the neck, filled with heparinized saline solution and daily flushed to prevent clotting. After a week to recover from surgery, the animals were connected to a HP 1290A pressure transducer connected to an HP 8805B carrier amplifier of a Hewlett Packard HP 7700 multichannel polygraph, in order to monitor the arterial blood pressure. Heart rate was manually computed from the pressure trace.

All animals were alternatively treated with placebo, lercanidipine and its (R)- and (S)-enantiomers. The drugs were administered by the oral route with a straight round aseptic tip catheter (Pores Serlat - France). The drugs were suspended in aqueous 0.5% Methocel A4C plus Antifoam M10 (10%). The administered volume was 1 ml/kg. The suspending medicine was used as placebo. During experimental performance, the arterial blood pressure was continuously recorded 30 minutes before (Basal Values) and up to 8 hours after drug administration.

Lercanidipine and (S)-lercanidipine induced a dose-related decrease in arterial blood pressure. The ED₂₅ values (dose inducing 25% decrease in DBP at peak effect) were evaluated by linear regression analysis and are summarized in Table 4.

**TABLE 4**

| Compound | ED₂₅ (mg/kg) 95% C.L. |
|---|---|
| Lercanidipine | 0.9 (0.5 ö 1.6) |
| (S)-Lercanidipine | 0.4 (0.3 ö 0.7) |
| (R)-Lercanidipine | > > 30 |
| C.L. = Confidence limits (S)-Lercanidipine exerted the most potent antihypertensive action, proving to be two fold more efficacious than the racemate, whereas the (R)-enantiomer did not affect blood pressure (less than 10% decrease in DBP) up to 30 mg/kg. | |

## Claims

1. Use of a compound of the general formula I 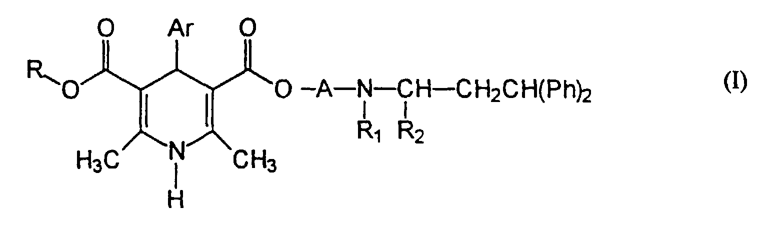 wherein
Ph represents a phenyl group,
Ar represents a 2-nitrophenyl, 3-nitrophenyl, 2,3-dichlorophenyl or benzofurazan-4-yl group,
A represents a branched chain alkylene group having from 3 to 6 carbon atoms,
R represents a straight or branched chain alkyl group having from 1 to 6 carbon atoms, optionally mono-substituted by an alkoxy group having from 1 to 6 carbon atoms,
R₁ represents a hydrogen atom, a hydroxy group or an alkyl group having from 1 to 4 carbon atoms, and
R₂ represents a hydrogen atom or a methyl group,
or of a salt, enantiomer, hydrate or solvate of such a compound, for the preparation of a medicament for the prevention, arrest or reversal of atherosclerotic degradation in the arterial walls of a patient.

2. Use of lercanidipine, (S)-lercanidipine or (R)-lercanidipine, or of a salt, hydrate or solvate of such a compound, for the preparation of a medicament for the prevention, arrest or reversal of atherosclerotic degradation in the arterial walls of a patient.

3. Use according to claim 1 or claim 2 for the preparation of a medicament which contains a pharmaceutically acceptable carrier.

4. Use according to any preceding claim for the preparation of a medicament in a form suitable for oral administration.

5. Use according to claim 4 for the preparation of a medicament which contains from 5% to 70% of the compound.

6. Use according to claim 4 or claim 5 for the preparation of a medicament which contains from 0.1 mg to 400 mg of the compound in single dose form.

7. Use according to claim 4 or claim 5 for the preparation of a medicament which contains from 1 mg to 200 mg of the compound in single dose form.

8. Use according to any of claims 1 to 3 for the preparation of a medicament in a form suitable for parenteral administration.

9. Use according to claim 8 for the preparation of a medicament which contains from 0.5% to 30% of the compound.

10. Use according to claim 8 or claim 9 for the preparation of a medicament which contains from 0.5 mg to 100 mg of the compound in single dose form.

## Patentansprüche

1. Verwendung einer Verbindung der allgemeinen Formel I 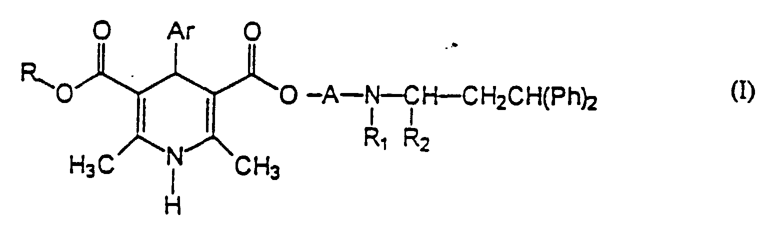 in der
Ph eine Phenylgruppe bedeutet,
Ar eine 2-Nitrophenyl-, 3-Nitrophenyl-, 2,3-Dichlorphenyl- oder Benzofurazan-4-yl-Gruppe bedeutet,
A einen verzweigtkettigen Alkylenrest mit 3 bis 6 Kohlenstoffatomen bedeutet,
R einen gerad- oder verzweigtkettigen Alkylrest mit 1 bis 6 Kohlenstoffatomen bedeutet, der gegebenenfalls durch einen Alkoxyrest mit 1 bis 6 Kohlenstoffatomen einfach substituiert ist,
R₁ ein Wasserstoffatom, eine Hydroxygruppe oder einen Alkylrest mit 1 bis 4 Kohlenstoffatomen bedeutet und
R₂ ein Wasserstoffatom oder eine Methylgruppe bedeutet,
oder eines Salzes, Enantiomers, Hydrats oder Solvats einer solchen Verbindung zur Herstellung eines Medikamentes zum Verhindern, Aufhalten oder Rückgängigmachen von atherosklerotischer Degeneration in den Arterienwanden eines Patienten.

2. Verwendung von Lercanidipin, (S)-Lercanidipin oder (R)-Lercanidipin oder eines Salzes, Hydrats oder Solvats einer solchen Verbindung zur Herstellung eines Medikamentes zum Verhindern, Aufhalten oder Rückgängigmachen von atherosklerotischer Degeneration in den Arterienwänden eines Patienten.

3. Verwendung nach Anspruch 1 oder Anspruch 2 zur Herstellung eines Medikamentes, das einen pharmazeutisch verträglichen Träger enthält.

4. Verwendung nach einem der vorstehenden Ansprüche zur Herstellung eines Medikamentes in einer zur oralen Verabreichung geeigneten Form.

5. Verwendung nach Anspruch 4 zur Herstellung eines Medikamentes, das 5% bis 70% der Verbindung enthält.

6. Verwendung nach Anspruch 4 oder Anspruch 5 zur Herstellung eines Medikamentes, das 0,1 mg bis 400 mg der Verbindung in Einzeldosisform enthält.

7. Verwendung nach Anspruch 4 oder Anspruch 5 zur Herstellung eines Medikamentes, das 1 mg bis 200 mg der Verbindung in Einzeldosisform enthält.

8. Verwendung nach einem der Ansprüche 1 bis 3 zur Herstellung eines Medikamentes in einer zur parenteralen Verabreichung geeigneten Form.

9. Verwendung nach Anspruch 8 zur Herstellung eines Medikamentes, das 0,5% bis 30% der Verbindung enthält.

10. Verwendung nach Anspruch 8 oder Anspruch 9 zur Herstellung eines Medikamentes, das 0,5 mg bis 100 mg der Verbindung in Einzeldosisform enthält.

## Revendications

1. Utilisation d'un composé de formule générale I 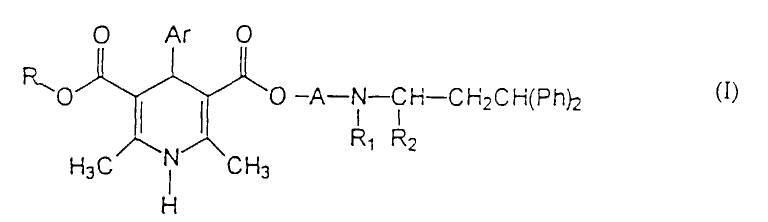 dans laquelle
Ph représente un groupe phényle,
Ar représente un groupe 2-nitrophényle, 3-nitrophényle, 2,3-dichlorophényle ou benzofurazan-4-yle,
A représente un groupe alkylène à chaîne ramifiée ayant de 3 à 6 atomes de carbone,
R représente un groupe alkyle à chaîne linéaire ou ramifiée ayant de 1 à 6 atomes de carbone, éventuellement monosubstitué par un groupe alcoxy ayant de 1 à 6 atomes de carbone,
R₁ représente un atome d'hydrogène, un groupe hydroxy ou un groupe alkyle ayant de 1 à 4 atomes de carbone, et
R₂ représente un atome d'hydrogène ou un groupe méthyle,
ou d'un sel, d'un énantiomère, d'un produit hydraté ou de solvatation d'un tel composé,
pour la préparation d'un médicament pour la prévention, l'arrêt ou l'inversion d'une dégradation athéroscléreuse des parois artérielles d'un patient.

2. Utilisation de la lercanidipine, de la (S)-lercanidipine ou de la (R)-lercanidipine, ou d'un sel, d'un produit hydraté ou de solvatation d'un tel composé, pour la préparation d'un médicament pour la prévention, l'arrêt ou l'inversion d'une dégradation athéroscléreuse des parois artérielles d'un patient.

3. Utilisation selon la revendication 1 ou la revendication 2, pour la préparation d'un médicament qui contient un véhicule pharmaceutiquement acceptable.

4. Utilisation selon l'une quelconque des revendications précédentes, pour la préparation d'un médicament sous une forme appropriée pour une administration orale.

5. Utilisation selon la revendication 4, pour la préparation d'un médicament qui contient de 5 % à 70 % du composé.

6. Utilisation selon la revendication 4 ou la revendication 5, pour la préparation d'un médicament qui contient de 0,1 mg à 400 mg du composé sous forme de dose unique.

7. Utilisation selon la revendication 4 ou la revendication 5, pour la préparation d'un médicament qui contient de 1 mg à 200 mg du composé sous forme de dose unique.

8. Utilisation selon l'une quelconque des revendications 1 à 3, pour la préparation d'un médicament sous une forme appropriée pour une administration parentérale.

9. Utilisation selon la revendication 8, pour la préparation d'un médicament qui contient de 0,5 % à 30 % du composé.

10. Utilisation selon la revendication 8 ou la revendication 9, pour la préparation d'un médicament qui contient de 0,5 mg à 100 mg du composé sous forme de dose unique.
